# EUROPÄISCHE PATENTSCHRIFT

(11) **EP 2 000 117 B1**
(45) Veröffentlichungstag und Bekanntmachung des Hinweises auf die Patenterteilung: **13.10.2010**
(21) Anmeldenummer: 08008084.9
(22) Anmeldetag: 26.04.2008
(51) Int. Cl.: A61F 2/46, B26D 1/00, B26D 1/30, B26D 5/02, B26D 7/02, B26D 7/26

(54) **Vorrichtung zum Präparieren von Gewebescheiben, insbesondere Knorpelscheiben**
Device for preparing tissue discs, in particular cartilage discs
Dispositif de préparation de disques de tissus, en particulier des disques de cartilage

(30) Priorität: 08.06.2007 DE 102007026574
(43) Veröffentlichungstag der Anmeldung: 10.12.2008
(73) Patentinhaber: Karl Storz GmbH & Co. KG, 78532 Tuttlingen (DE)
(72) Erfinder: Eisenkolb, Peter, 78532 Tuttlingen (DE)
(74) Vertreter: Hofmeister, Frank

(56) Entgegenhaltungen:
- WO-A-2008/008240
- US-A- 4 228 707
- US-A1- 2003 125 811

## Beschreibung

Die Erfindung betrifft eine Vorrichtung zum Präparieren von Gewebescheiben, insbesondere Knorpelscheiben.

Zum Präparieren von Gewebescheiben, beispielsweise zum Schaffen einer neuen Nasenscheidewand aus einem explantierten Zwischenrippenstück, ist es erforderlich, die zu schaffende Plastik (Gewebescheibe) auf eine vorbestimmte Stärke zu präparieren. In der Praxis erfolgt dieses Präparieren von Hand mittels eines Skalpells. Neben dem Umstand, dass dieses praktizierte Präparieren mittels des Skalpells ein hohes Verletzungsrisiko für den Anwender birgt, ist es äußerst schwierig, exakte Abmessungen der Plastik, insbesondere bezüglich der Dicke der Gewebescheibe, zu gewährleisten.

Eine Vorrichtung zum Präpariren von Gewebe gemäß der Präambel von Anspruch 1 ist offenbart in US 2003/0125811.

Davon ausgehend liegt der Erfindung die Aufgabe zugrunde, eine Vorrichtung zum Präparieren von Gewebescheiben zu schaffen, die bei einfachem Aufbau eine sichere und exakte Handhabung gewährleistet.

Die Lösung dieser Aufgabenstellung wird erfindungsgemäß durch eine Vorrichtung nach Anspruch 1 erzielt.

Durch die erfindungsgemäße Kombination einer Klemmvorrichtung und einer Schneidvorrichtung in einer Apparatur ist erstmalig möglich Gewebescheiben vorgegebener Größe und Dicke schnell und exakt zu präparieren. Auf die aus der Praxis bekannte Verwendung eines Skalpells wird bei der erfindungsgemäßen Vorrichtung ganz verzichtet.

Um den mittels der Klemmvorrichtung zu fixierenden Gewebeblock einerseits nicht zu stark zu quetschen, andererseits den Gewebeblock aber auch sicher zu fixieren, wird vorgeschlagen, dass im geschlossenen Zustand der Klemmvorrichtung zwischen den mindestens zwei Klemmbacken der Klemmvorrichtung ein Klemmspalt verbleibt, dessen Höhe vorteilhafterweise verstellbar ist, so dass die Vorrichtung für verschiede Gewebescheibengrößen verwendbar ist.

Die starre Klemmbacke ist vorteilhafterweise als doppelwandige Platte mit einer fensterartigen Öffnung so ausgebildet, dass die gegenüber der starren Klemmbacke verschwenkbare Klemmbacke innerhalb eines zwischen den beiden voneinander beabstandeten Seitenwänden der doppelwandigen Platte ausgebildeten Zwischenraums führend gelagert ist. Diese Anordnung der verschwenkbaren Klemmbacke innerhalb des Zwischenraums der starren Klemmbacke gewährleistet eine exakte und verkantungsfreie Führung der verschwenkbaren Klemmbacke.

Die Dicke der zu präparierenden Gewebescheibe ist vorteilhafterweise dadurch variierbar, dass die Breite des durch die Seitenwände der doppelwandigen Platte gebildeten Zwischenraums einstellbar ist.

Zur Lagerung der verschwenkbaren Klemmbacke wird vorgeschlagen, dass diese einseitig in der Platte der starren Klemmbacke gelagert ist, wobei die Höhe des zwischen der starren und der verschwenkbaren Klemmbacke verbleibenden Klemmspalts über eine Verlagerung des Lagerpunktes der verschwenkbaren Klemmbacke in der Platte einstellbar ist.

Um den zu präparierenden Gewebeblock sicher und lagestabil in dem Klemmspalt halten zu können, wird vorgeschlagen, dass zumindest an den einander zugewandten Flächen der mindestens zwei Klemmbacken zahnartige Fixierungselemente ausgebildet sind.

Um eine immer gleichbleibende Schnittqualität gewährleisten zu können wird weiterhin vorgeschlagen, dass die mindestens eine Schneidkante auswechselbar am Schwenkarm angeordnet ist. Die Auswechselbarkeit der Schneidkante hat weiterhin den Vorteil, dass nur die dem direkten Verschleiß unterliegende Schneidkante ausgetauscht werden muss, nicht aber der gesamte Schwenkarm.

Schließlich wird vorgeschlagen, dass der Abstand der beiden parallelen Armteile und somit auch der beiden Schneidkanten zueinander einstellbar ist. Über diese Variation des Schneidkantenabstandes zueinander lässt sich die Dicke der zu präparierenden Gewebescheibe einfach und zuverlässig einstellen und einhalten.

Weitere Merkmale und Vorteile der Erfindung ergeben sich anhand der zugehörigen Zeichnung, in der ein Ausführungsbeispiel einer erfindungsgemäßen Vorrichtung zum Präparieren von Gewebescheiben, insbesondere Knorpelscheiben, nur beispielhaft dargestellt ist, ohne die Erfindung auf dieses Ausführungsbeispiel zu beschränken. In der Zeichnung zeigt:
- Fig. 1: eine perspektivische Seitenansicht einer erfindungsgemäßen Vorrichtung zum Präparieren von Gewebescheiben, eine erste Arbeitsstellung darstellend;
- Fig. 2: eine Ansicht gemäß Fig. 1, jedoch eine zweite Arbeitsstellung darstellend und
- Fig. 3: eine um 180° gedrehte Ansicht gemäß Fig. 1.

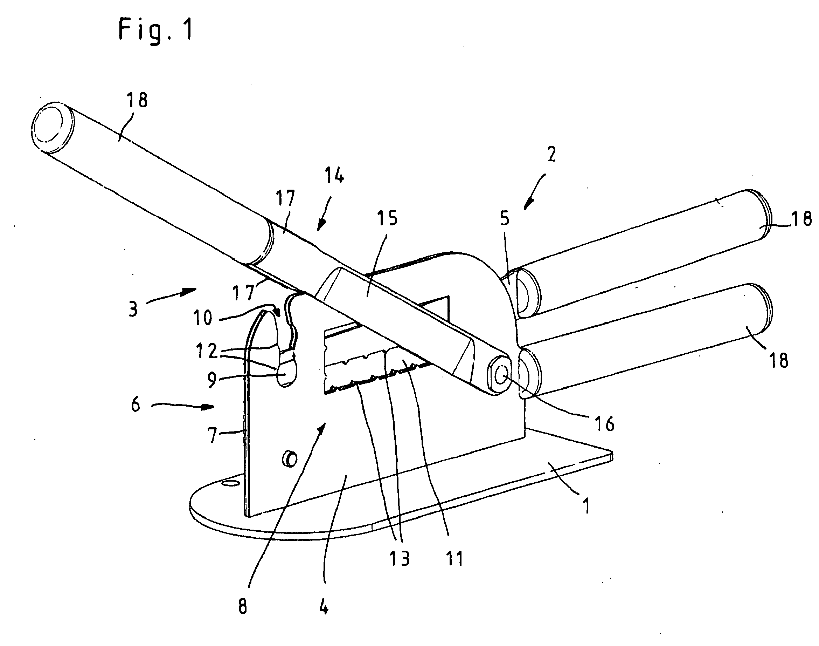

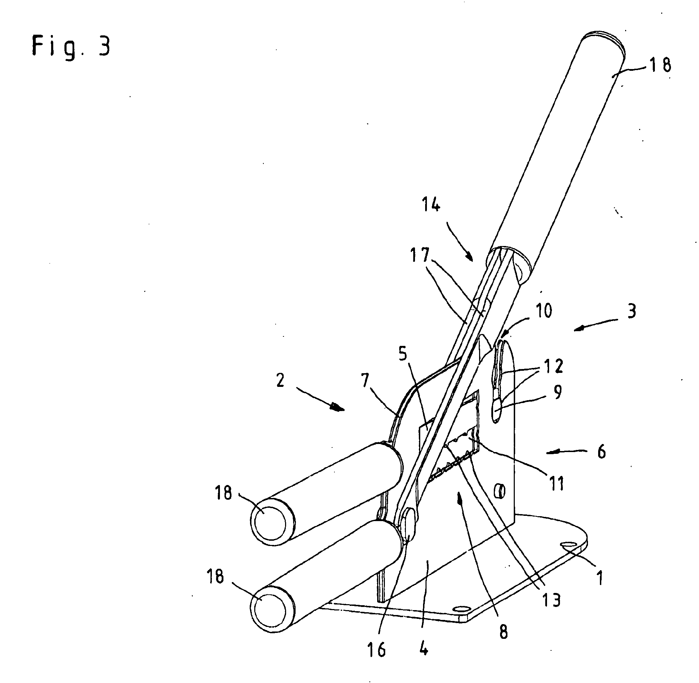

Die in den Abbildungen Fig. 1 bis 3 dargestellte Vorrichtung zum Präparieren von Gewebescheiben, insbesondere Knorpelscheiben, besteht im Wesentlichen aus einem als Standfläche dienenden Grundkörper 1 sowie einer Klemmvorrichtung 2 und einer Schneidvorrichtung 3, die auf dem Grundkörper 1 angeordnet sind.

Eine derartige Vorrichtung dient zum Präparieren von Gewebescheiben, beispielsweise zum Schaffen einer neuen Nasenscheidewand aus einem explantierten Zwischenrippenstück. Selbstverständlich ist es auch möglich, andere, gegebenenfalls auch künstliche Gewebe mit dieser Vorrichtung zu bearbeiten.

Bei der dargestellten Ausführungsform besteht die Klemmvorrichtung 2 aus einer starren Klemmbacke 4 und einer gegenüber der starren Klemmbacke 4 verschwenkbaren Klemmbacke 5. Wie aus den Abbildungen ersichtlich, ist die starre Klemmbacke 4 als doppelwandige Platte 6 so ausgebildet, dass beide Seitenwände einen Zwischenraum 7 ausbildend voneinander beabstandet sind. Zur Aufnahme eines zu präparierenden Gewebeblocks ist in der Platte 6 eine fensterartige Öffnung 8 ausgebildet.

Vervollständigt wird die Klemmvorrichtung 2 durch die verschwenkbare Klemmbacke 5, die, wie aus den Abbildungen ersichtlich, in dem von der doppelwandigen Platte 6 der starren Klemmbacke 4 gebildeten Zwischenraum 7 führend gelagert ist, wobei die eigentliche verschwenkbare Lagerung der verschwenkbaren Klemmbacke 5 über einen an einem Ende der Klemmbacke 5 angeordneten Lagerzapfen 9 erfolgt, der in eine in der Platte 6 der starren Klemmbacke 4 ausgebildete Lageraufnahme 10 einsetzbar ist.

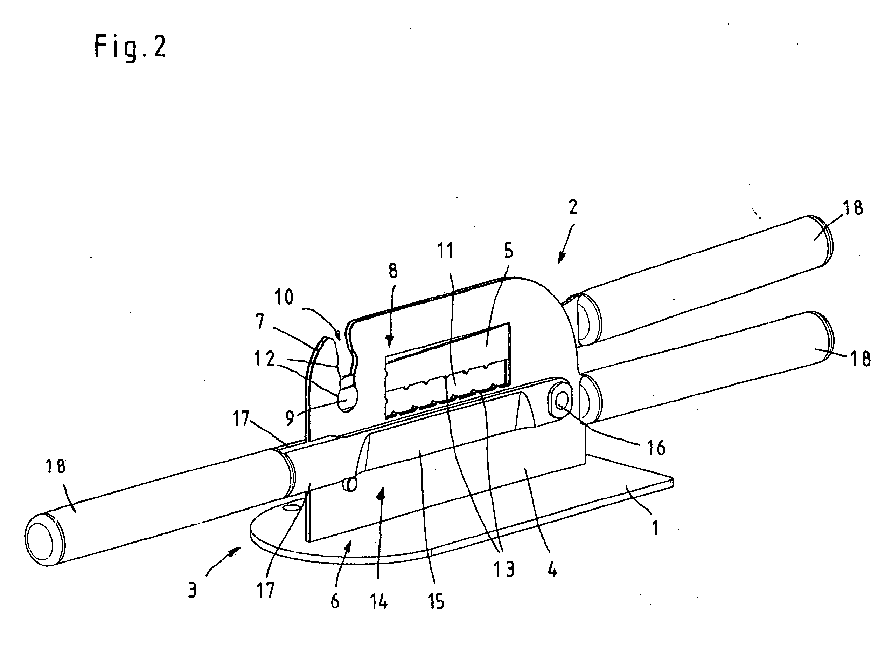

Wie insbesondere aus Fig. 2 ersichtlich, ist die Klemmvorrichtung 2 so ausgebildet, dass auch im geschlossenen Zustand der Klemmvorrichtung 2, das heißt, wenn die verschwenkbare Klemmbacke 5 die fensterartige Öffnung 8 teilweise verschlie-ßend abwärts verschwenkt wurde, zwischen der verschwenkbaren Klemmbacke 5 und der Oberkante der starren Klemmbacke 4 ein offener Klemmspalt 11 zur klemmenden Aufnahme der zu präparierenden Gewebescheibe verbleibt.

Bei der dargestellten Ausführungsform ist der Lagerzapfen 9 der verschwenkbaren Klemmbacke 5 unrund so ausgebildet, dass die verschenkbare Klemmbacke 5 nur in einer im Wesentlichen senkrecht zum Grundkörper 1 ausgerichteten Position in die Lageraufnahme 10 einsetzbar ist und in der Lageraufnahme 10 einzelne Lagerpunkte 12 ausgebildet sind, in denen die verschwenkbare Klemmbacke 5 zum Schließen der Klemmvorrichtung 2 abwärts in Richtung der fensterartigen Öffnung 8 der Platte 6 verschwenkbar ist. Um die Höhe des Klemmspalts 11 zur Aufnahme größerer oder kleinerer Gewebeblöcke variieren zu können, sind in der Lageraufnahme 10 mehrere über- bzw. untereinander angeordnete Lagerpunkte 12 zur verschwenkbaren Lagerung der verschwenkbaren Klemmbacke 5 ausgebildet.

Weiterhin weist die Klemmvorrichtung 2 zumindest an den einander zugewandten Flächen der starren Klemmbacke 4 sowie der verschwenkbaren Klemmbacke 5 zahnartige Fixierungselemente 13 auf, die einen sichern und lagestabilen Halt des in der Klemmvorrichtung 2 angeordneten, zu bearbeitenden Gewebeblocks gewährleisten.

Die Schneidvorrichtung 3 besteht bei der dargestellten Ausführungsform aus einem gegenüber der Klemmvorrichtung 2 verschwenkbar ausgebildeten Schwenkarm 14, der zum Schneiden des in der Klemmvorrichtung 2 gehaltenen Gewebeblocks mindestens eine Schneidkante 15 aufweist, wobei der Schwenkarm 14 bei der dargestellten Ausführungsform verschwenkbar auf einem an der Platte 6 angeformten Lagerzapfen 16 gelagert ist. Alternativ ist es selbstverständlich auch möglich, den Schwenkarm 14 am Grundkörper 1 zu lagern.

Um eine immer gleichbleibende Schnittqualität gewährleisten zu können, ist die mindestens eine Schneidkante 15 vorzugsweise auswechselbar am Schwenkarm 14 angeordnet.

Bei der dargestellten Ausführungsform weist der Schwenkarm 14 zwei parallel zueinanderverlaufende Armteile 17 auf, die jeweils mit einer Schneidkante 15 versehen so an der Platte 6 gelagert sind, dass die Platte 6 zwischen den beiden Armteilen 17 des Schwenkarms 14 angeordnet ist. Diese Ausgestaltung der Schneidvorrichtung 3 ermöglicht das gleichzeitige Ausführen zweier paralleler Schnitte zum Präparieren einer Gewebescheibe aus dem in der Klemmvorrichtung 2 gehaltenen Gewebeblock.

Um verschieden dicke Gewebescheiben mittels der Schneidvorrichtung 3 erstellen zu können, bestehen zur Veränderung des Abstandes zwischen den beiden Armteilen 17 des Schwenkarms 14 der Schneidvorrichtung 3 zwei Möglichkeiten, nämlich einerseits die Veränderung der Breite des zwischen den Seitenwänden der doppelwandigen Platte 6 ausgebildeten Zwischenraums 7 durch Erweiterung oder Verengung des Seitenwandabstandes der Platte 6 und andererseits die Veränderung des Abstandes zwischen den beiden Armteilen 17 des Schwenkarms 14 der Schneidvorrichtung 3 bei unveränderter Breite des zwischen den Seitenwänden der doppelwandigen Platte 6 ausgebildeten Zwischenraums 7.

Alternativ zu der dargestellten Ausbildung der Schneidvorrichtung 3 mit einem verschwenkbar gelagerten Schwenkarm 14 ist es selbstverständlich auch möglich, die Schneidvorrichtung 3 so auszugestalten, dass die Schneidvorrichtung 3 mindestens einen gegenüber der Klemmvorrichtung 2 axial verschiebbaren, mit mindestens einer Schneidkante 15 versehener Schneidarm aufweist. Die Axialverschiebbarkeit des Schneidarms gegenüber der Klemmvorrichtung 2 ermöglicht die Ausübung einer sägenden Schneidbewegung der Schneidvorrichtung 3.

Der Zusammenbau sowie die Handhabung der zuvor beschriebenen Vorrichtung zum Präparieren von Gewebescheiben geschieht wie folgt:

Vor der Benutzung der Vorrichtung werden zunächst die verschwenkbare Klemmbacke 5 und der Schwenkarm 14 der Schneidvorrichtung 3 mit der Vorrichtung verbunden. Hierzu wird die verschwenkbare Klemmbacke 5 in einer im Wesentlichen senkrecht zum Grundkörper 1 ausgerichteten Stellung mit dem Lagerzapfen 9 voran in die in der doppelwandigen Platte 6 ausgebildete Lageraufnahme eingesteckt, bis der Lagerzapfen 9 den Lagerpunkt 12, der der für den jeweiligen Einsatzzweck erforderlichen Höhe des Klemmspalts 11 entspricht.

Danach kann die verschwenkbare Klemmbacke 5 um 90° nach unten verschwenkt werden, bis die jeweilige minimale Höhe des Klemmspalts 11 erreicht ist.

Nachfolgend wird der Schwenkarm 14 der Schneidvorrichtung auf den an der Platte 6 ausgebildeten Lagerzapfen 16 aufgesetzt, wozu am vorderen Ende des Schwenkarms 14 eine mit der Kontur des Lagerzapfens 16 korrespondierende Lageraufnahme ausgebildet ist. Um ein versehentliches Trennen des Schwenkarms 14 vom Lagerzapfen 16 zu verhindern, ist der Lagerzapfen 16 unrund ausgebildet, so dass der Schwenkarm 14 nur in einer Position, nämlich im Wesentlichen senkrecht zum Grundkörper 1, auf den Lagerzapfen 16 aufsteckbar und von diesem wieder trennbar ist. In allen anderen Positionen erlaubt die Lagerung auf dem Lagerzapfen 16 nur ein verschwenken des Schwenkarms 14 um den Lagerzapfen 16.

Um dem Schwenkarm 14 sowie der verschwenkbaren Klemmbacke 5 einen größtmöglichen Verschwenkbereich einzuräumen, sind die Lagerstellen der Bauteile 5 und 14 einander gegenüberliegend an der Platte 6 angeordnet.

Alternativ ist es jedoch auch möglich, die Lagerstellen der Bauteile 5 und 14 auf einer Seite der Platte 6 anzuordnen und den Schwenkarm 14 beispielsweise als die komplette Klemmvorrichtung 2 umspannend zweigeteilt auszugestalten.

Zur bequemeren Handhabung der Vorrichtung sind an den den Lagerenden entgegengesetzten Enden sowohl der veschwenkbaren Klemmbacke 5 als auch des Schwenkarms 14 der Schneidvorrichtung 3 Handgriffe 18 angeformt, über die sich die Bauteile 5 und 14 einfach verschwenken lassen. Zusätzlich weist die dargestellte Vorrichtung einen an der Platte 6 der starren Klemmbacke 4 angeformten Handgriff 18 auf, um einerseits durch Ergreifen der Handgriffe 18 beider Klemmbacken 4 und 5 eine ausreichende Klemmkraft aufbieten zu können und andererseits den Grundkörper 1 samt starrer Klemmbacke 4 einfach und sicher transportieren zu können.

Nach dem Montieren der verschwenkbaren Klemmbacke 5 sowie des Schwenkarms 14 ist die Vorrichtung nunmehr einsatzbereit.

Zum Einfügen eines zu bearbeitenden Gewebeblocks in die Klemmvorrichtung 2 werden zunächst die verschwenkbare Klemmbacke 5 und der Schwenkarm 14 der Schneidvorrichtung 3 soweit nach oben in Richtung der senkrechten Position verschwenkt, bis diese gegeneinander anlaufen. Anschließend wird beispielsweise ein explantiertes Knorpelstück oder ein anderes Gewebestück in die fensterartige Öffnung 8 der Platte 6 eingesetzt und durch Absenken der verschwenkbaren Klemmbacke 5 sicher und lagestabil eingeklemmt, wobei die zahnartigen Fixierungselemente 13 einen zusätzlichen Halt gewährleisten.

Das eigentliche Freipräparieren der Gewebescheibe erfolgt im nachfolgenden Arbeitsschritt durch das Absenken des Schwenkarms 14. Die beiden an den parallelen Armteilen 17 angeordneten Schneidkanten 15 führen beim Absenken des Schwenkarms 14 gleichzeitig zwei parallele Schnitte aus und erstellen so in einem Arbeitsgang die benötigte Gewebescheibe.

Die Breite der zu präparierenden Gewebescheibe lässt sich entweder durch die Veränderung der Breite des zwischen den Seitenwänden der doppelwandigen Platte 6 ausgebildeten Zwischenraums 7 oder durch die Veränderung des Abstandes zwischen den beiden Armteilen 17 des Schwenkarms 14 der Schneidvorrichtung 3 bewirken. Die Veränderung dieser Abstände kann beispielsweise über Gewinde erfolgen, wobei die Veränderung dieser Abstände mit einer Skalierung versehen sein kann und über die Handgriffe 18 oder einen oder mehrere zusätzliche Handgriffe, Stellschrauben, Drehknöpfe oder dergleichen einstellbar ist.

Dabei ist es zum einen möglich, nur den Abstand der beiden Schneidkanten 15 zueinander einzustellen, zum anderen aber auch, zusätzlich dazu den Zwischenraum 7 zwischen den Seitenwänden der doppelwandigen Platte 6 einzustellen, das heißt also den Abstand der beiden Seitenwände der doppelwandigen Platte 6 zueinander.

Neben dem einfachen Aufbau zeichnet sich eine solchermaßen aufgebaute Vorrichtung zum Präparieren von Gewebescheiben durch ihre sichere und exakte Handhabung aus.

### Bezugszeichenliste

- 1: Grundkörper
- 2: Klemmvorrichtung
- 3: Schneidvorrichtung
- 4: starre Klemmbacke
- 5: verschwenkbare Klemmbacke
- 6: Platte
- 7: Zwischenraum
- 8: Öffnung
- 9: Lagerzapfen
- 10: Lageraufnahme
- 11: Klemmspalt
- 12: Lagerpunkt
- 13: Fixierungselement
- 14: Schwenkarm
- 15: Schneidkante
- 16: Lagerzapfen
- 17: Armteil
- 18: Handgriff

## Patentansprüche

1. Vorrichtung zum Präparieren von Gewebescheiben, insbesondere Knorpelscheiben, mit einer Klemmvorrichtung (2) zum klemmenden Festlegen eines Gewebeblocks und mit mindestens einer Schneidvorrichtung (3) zum Bearbeiten des in der Klemmvorrichtung (2) gehaltenen Gewebeblocks, wobei die Klemmvorrichtung (2) eine auf einem Grundkörper (1) gelagerte starre Klemmbacke (4) und eine gegenüber der starren Klemmbacke (4) verschwenkbare Klemmbacke (5) aufweist,
**dadurch gekennzeichnet,**
**dass** die starre Klemmbacke (4) als doppelwandige Platte (6) ausgebildet ist und, dass die Schneidvorrichtung (3) als am Grundkörper (1) oder an der Platte (6) der starren Klemmbacke (4) gelagerter gegenüber der Klemmvorrichtung (2) verlagerbarer und mit mindestens einer Schneidkante (15) versehener Schwenkarm (15) ausgebildet ist, wobei der Schwenkarm (14) zumindest im Bereich der mindestens einen Schneidkante (15) zwei parallel zueinander verlaufende und jeweils mit einer Schneidkante (15) versehene Armteile (17) aufweist, die so am Grundköper (1) oder an der Platte (6) der starren Klemmbacke (4) gelagert sind, dass die Platte (6) zwischen den beiden Armteilen (17) angeordnet ist.

2. Vorrichtung nach Anspruch 1, **dadurch gekennzeichnet, dass** der Abstand der beiden parallelen Armteile (17) zueinander einstellbar ist.

3. Vorrichtung nach Anspruch 1 oder 2, **dadurch gekennzeichnet, dass** die mindestens eine Schneidkante (15) auswechselbar am Schwenkarm (14) angeordnet ist.

4. Vorrichtung nach einem der Ansprüche 1 bis 3, **dadurch gekennzeichnet, dass** im geschlossenen Zustand der Klemmvorrichtung (2) zwischen der starren Klemmbacke (4) und der verschwenkbaren Klemmbacke (5) der Klemmvorrichtung (2) ein Klemmspalt (11) verbleibt.

5. Vorrichtung nach Anspruch 4, **dadurch gekennzeichnet, dass** die Höhe des Klemmspalts (11) einstellbar ist.

6. Vorrichtung nach einem der Ansprüche 1 bis 5, **dadurch gekennzeichnet, dass** die doppelwandige Platte (6) der starren Klemmbacke (4) mit einer fensterartigen Öffnung (8) ausgebildet ist und die gegenüber der starren Klemmbacke (4) verschwenkbare Klemmbacke (5) innerhalb eines zwischen den beiden voneinander beabstandeten Seitenwänden der doppelwandigen Platte (6) ausgebildeten Zwischenraums (7) führend gelagert ist.

7. Vorrichtung nach Anspruch 6, **dadurch gekennzeichnet, dass** die Breite des durch die Seitenwände der doppelwandigen Platte (6) gebildeten Zwischenraums (7) einstellbar ist.

8. Vorrichtung nach Anspruch 6 oder 7, **dadurch gekennzeichnet, dass** die verschwenkbare Klemmbacke (5) einseitig in der Platte (6) der starren Klemmbacke (4) gelagert ist.

9. Vorrichtung nach einem der Ansprüche 4 bis 8, **dadurch gekennzeichnet, dass** die Höhe des Klemmspalts (11) über eine Verlagerung des Lagerpunktes der verschwenkbaren Klemmbacke (5) in der Platte (6) einstellbar ist.

10. Vorrichtung nach einem der Ansprüche 1 bis 9, **dadurch gekennzeichnet, dass** zumindest an den einander zugewandten Flächen der Klemmbacken (4, 5) zahnartige Fixierungselemente (13) ausgebildet sind.

## Claims

1. Device for preparing tissue discs, in particular cartilage discs, with a clamping device (2) for securing a tissue block by clamping, and with at least one cutting device (3) for working the tissue block held in the clamping device (2), said clamping device (2) having a rigid clamping jaw (4), mounted on a base (1), and a clamping jaw (5) that can be pivoted with respect to the rigid clamping jaw (4), **characterized in that** the rigid clamping jaw (4) is configured as a double-walled plate (6), and **in that** the cutting device (3) is configured as a pivot arm (14) that is mounted on the base (1) or on the plate (6) of the rigid clamping jaw (4) so as to be movable with respect to the clamping device (2) and that is provided with at least one cutting edge (15), which pivot arm (14), at least in the area of the at least one cutting edge (15), has two arm parts (17) running parallel to each other and each provided with a cutting edge (15), which arm parts (17) are mounted on the base (1) or on the plate (6) of the rigid clamping jaw (4) in such a way that the plate (6) is arranged between the two arm parts (17).

2. Device according to Claim 1, **characterized in that** the distance of the two parallel arm parts (17) from each other is adjustable.

3. Device according to Claim 1 or 2, **characterized in that** the at least one cutting edge (15) is arranged exchangeably on the pivot arm (14).

4. Device according to one of Claims 1 to 3, **characterized in that**, in the closed state of the clamping device (2), a clamping gap (11) remains between the rigid clamping jaw (4) and the pivotable clamping jaw (5) of the clamping device (2).

5. Device according to Claim 4, **characterized in that** the height of the clamping gap (11) is adjustable.

6. Device according to one of Claims 1 to 5, **characterized in that** the double-walled plate (6) of the rigid clamping jaw (4) is configured with a window-like opening (8), and the clamping jaw (5) that is pivotable with respect to the rigid clamping jaw (4) is mounted and guided inside a space (7) formed between the two spaced apart side walls of the double-walled plate (6).

7. Device according to Claim 6, **characterized in that** the width of the space (7) formed by the side walls of the double-walled plate (6) is adjustable.

8. Device according to Claim 6 or 7, **characterized in that** the pivotable clamping jaw (5) is mounted with one end in the plate (6) of the rigid clamping jaw (4).

9. Device according to one of Claims 4 to 8, **characterized in that** the height of the clamping gap (11) can be adjusted by shifting the bearing point of the pivotable clamping jaw (5) in the plate (6).

10. Device according to one of Claims 1 to 9, **characterized in that** tooth-like fixing elements (13) are formed at least on the mutually facing surfaces of the clamping jaws (4, 5).

## Revendications

1. Appareillage pour préparer des tranches de tissus, notamment des tranches de cartilage, comprenant un dispositif de serrage (2) pour assurer l'immobilisation par serrage d'un bloc de tissus, et comprenant également au moins un dispositif de coupe (3) pour travailler le bloc de tissus maintenu dans le dispositif de serrage (2), le dispositif de serrage (2) présentant un mors de serrage fixe (4) monté sur un corps de base (1) et un mors de serrage (5) pivotant par rapport au mors de serrage fixe (4),
**caractérisé en ce que** le mors de serrage fixe (4) est réalisé sous la forme d'une plaque (6) à double paroi, et **en ce que** le dispositif de coupe (3) est réalisé sous la forme d'un bras pivotant (14), qui est muni d'au moins un tranchant de coupe (15) et est monté sur le corps de base (1) ou sur la plaque (6) du mors de serrage fixe (4) en pouvant être déplacé par rapport au dispositif de serrage (2), le bras pivotant (14) présentant, au moins dans la zone dudit au moins un tranchant de coupe (15), deux parties de bras (17), qui s'étendent parallèlement l'une à l'autre et sont munies chacune d'un tranchant de coupe (15), et qui sont montées sur le corps de base (1) ou sur la plaque (6) du mors de serrage fixe (4) de façon telle, que la plaque (6) soit agencée entre les deux parties de bras (17).

2. Appareillage selon la revendication 1, **caractérisé en ce que** la distance d'espacement entre les deux parties de bras (17) parallèles, est réglable.

3. Appareillage selon la revendication 1 ou la revendication 2, **caractérisé en ce que** ledit au moins un tranchant de coupe (15) est agencé de manière interchangeable sur le bras pivotant (14).

4. Appareillage selon l'une des revendications 1 à 3, **caractérisé en ce que** dans l'état fermé du dispositif de serrage (2), il reste un interstice de serrage (11) entre le mors de serrage fixe (4) et le mors de serrage pivotant (5) du dispositif de serrage (2).

5. Appareillage selon la revendication 4, **caractérisé en ce que** la hauteur de l'interstice de serrage (11) est réglable.

6. Appareillage selon l'une des revendications 1 à 5, **caractérisé en ce que** la plaque (6) à double paroi du mors de serrage fixe (4) est réalisée avec une ouverture (8) en forme de fenêtre, et le mors de serrage (5) pivotant par rapport au mors de serrage fixe (4), est monté de manière à être guidé à l'intérieur d'un espace intermédiaire (7) formé entre les deux parois latérales, espacées l'une de l'autre, de la plaque (6) à double paroi.

7. Appareillage selon la revendication 6, **caractérisé en ce que** la largeur de l'espace intermédiaire (7) formé par les parois latérales de la plaque (6) à double paroi, est réglable.

8. Appareillage selon la revendication 6 ou la revendication 7, **caractérisé en ce que** le mors de serrage pivotant (5) est monté, d'un côté, dans un palier de pivotement dans la plaque (6) du mors de serrage fixe (4).

9. Appareillage selon l'une des revendications 4 à 8, **caractérisé en ce que** la hauteur de l'interstice de serrage (11) est réglable par déplacement du point de palier du mors de serrage pivotant (5) dans la plaque (6).

10. Appareillage selon l'une des revendications 1 à 9, **caractérisé en ce qu'**au moins sur les surfaces des mors de serrage (4, 5), qui sont dirigées l'une vers l'autre, sont réalisés des éléments d'arrêt (13) en forme de dents.
